# EUROPÄISCHE PATENTSCHRIFT

(11) **EP 2 419 221 B1**
(45) Veröffentlichungstag und Bekanntmachung des Hinweises auf die Patenterteilung: **29.05.2013**
(21) Anmeldenummer: 09777555.5
(22) Anmeldetag: 30.07.2009
(51) Int. Cl.: B05C 17/005, A45D 34/04, A46B 17/06

(54) **BEHÄLTNIS FÜR AUFTRAGSORGAN**
RECEPTACLE FOR AN APPLICATION MEANS
RÉCEPTACLE POUR DES MOYENS D'APPLICATION

(30) Priorität: 13.04.2009 WO PCT/EP2009/002810; 13.04.2009 WO PCT/EP2009/002807
(43) Veröffentlichungstag der Anmeldung: 22.02.2012
(73) Patentinhaber: Dadgar, Karan, 22765 Hamburg (DE)
(72) Erfinder: Dadgar, Karan, 22765 Hamburg (DE)
(74) Vertreter: Heun, Thomas
(86) Internationale Anmeldenummer: PCT/EP2009/005533
(87) Internationale Veröffentlichungsnummer: WO 2010/118765

(56) Entgegenhaltungen:
- EP-A2- 0 420 047
- WO-A1-03/084362
- DE-A1- 4 444 765

## Beschreibung

Die Erfindung betrifft ein Behältnis für ein Auftragsorgan für Fluide wie insbesondere flüchtige Lösungsmittel enthaltende Flüssigkeiten oder flüssige oder breiige Massen oder Pasten wie zum Beispiel Farbstoffe, Klebstoffe und Dichtungsmassen sowie Lebensmittelmassen wie Gewürzsaucen und Brotaufstriche usw., wobei das Auftragsorgan zum Beispiel eine Düse oder eine Kanüle, einen Pinsel, ein Schwamm-ähnliches oder ein anderes, für das Fluid saugfähige bzw. dieses abgebende Material aufweist und üblicherweise Teil einer Abgabeeinrichtung oder eines Auftragsgerätes (wie zum Beispiel einer spritzenartigen Vorrichtung, einer Injektionsspritze oder eines Spenders) ist. Das Behältnis dient dabei zur Aufnahme eines solchen Auftragsorgans, wenn es nicht benötigt bzw. nicht angewendet wird (siehe DE 44 44 765 A1).

Das Auftragen von Fluiden mittels eines Auftragsorgans der oben genannten Art kann Probleme verursachen, wenn bei Nichtgebrauch des Auftragsorgans das Fluid unter Einfluss der Umgebungsluft eintrocknet und dadurch die Funktion des Auftragsorgans beeinträchtigt. Bekannt ist dieses Problem insbesondere im Zusammenhang mit Fluiden, die flüchtige Lösungsmittel enthalten, wie insbesondere Farbstoffe, Lacke, Ölfarben und Klebstoffe, und zwar insbesondere nachdem die betreffende Abgabeeinrichtung oder das Auftragsgerät das erste Mal benutzt oder angebrochen wurde, da dieses dann nicht mehr zuverlässig und luftdicht verschlossen werden kann. Das Auftragsorgan kann dann schnell unbrauchbar werden, so dass es aufwändig gereinigt oder ausgetauscht werden muss.

Eine Aufgabe, die der Erfindung zugrunde liegt, besteht deshalb darin, nach einer Lösung für dieses Problem zu suchen und insbesondere ein Behältnis für ein solches Auftragsorgan zu schaffen, mit dem ein Eintrocknen des Fluids zumindest über eine sehr lange Zeit verhindert werden kann.

Gelöst wird diese Aufgabe gemäß Anspruch 1 mit einem Behältnis für ein Auftragsorgan einer Abgabeeinrichtung oder eines Auftragsgerätes für Fluide, zur Aufnahme des Auftragsorgans bei Nichtgebrauch, das sich auszeichnet durch ein Gehäuse mit einem Verbindungsteil zur zumindest im Wesentlichen luftdichten Verbindung des Behältnisses mit einem korrespondierenden Verbindungsteil an der Abgabeeinrichtung oder dem Auftragsgerät, mit einem porösen oder saugfähigen Material, das mit einem Verdünnungs- oder Lösungsmittel für das Fluid getränkt ist, und mit einem freien Raum oder einer Ausnehmung in dem porösen oder saugfähigen Material zur zumindest im Wesentlichen berührungsfreien Aufnahme des Auftragsorgans, wobei das Verdünnungs- oder Lösungsmittel so gewählt ist, dass es sich nach Verdampfung durch Niederschlag auf dem Auftragsorgan ablagert.

Ein besonderer Vorteil dieser Lösung besteht darin, dass durch die zumindest weitgehend luftdichte Befestigung des Behältnisses an der Abgabeeinrichtung in Verbindung mit dem in dem Gehäuse vorhandenen Verdünnungs- oder Lösungsmittel in besonders wirksamer Weise ein Aushärten des Fluids an dem Auftragsorgan über eine sehr lange Zeit verhindert werden kann.

Die Unteransprüche haben vorteilhafte Weiterbildungen der Erfindung zum Inhalt.

Weitere Einzelheiten, Merkmale und Vorteile der Erfindung ergeben sich aus der folgenden Beschreibung von bevorzugten Ausführungsformen anhand der Zeichnung. Es zeigt:
- Fig. 1: eine erste Ausführungsform eines erfindungsgemäßen Behältnisses zusammen mit einem dazu passenden Auftragsorgan eines Auftragsgerätes;
- Fig. 2: eine zweite Ausführungsform eines erfindungsgemäßen Behältnisses zusammen mit einem dazu passenden Auftragsorgan eines Auftragsgerätes;
- Fig. 3: eine Detaildarstellung des Behältnisses gemäß Figur 2; und
- Fig. 4: eine dritte Ausführungsform eines erfindungsgemäßen Behältnisses zusammen mit einem dazu passenden Auftragsorgan in Form eines Pinsels.

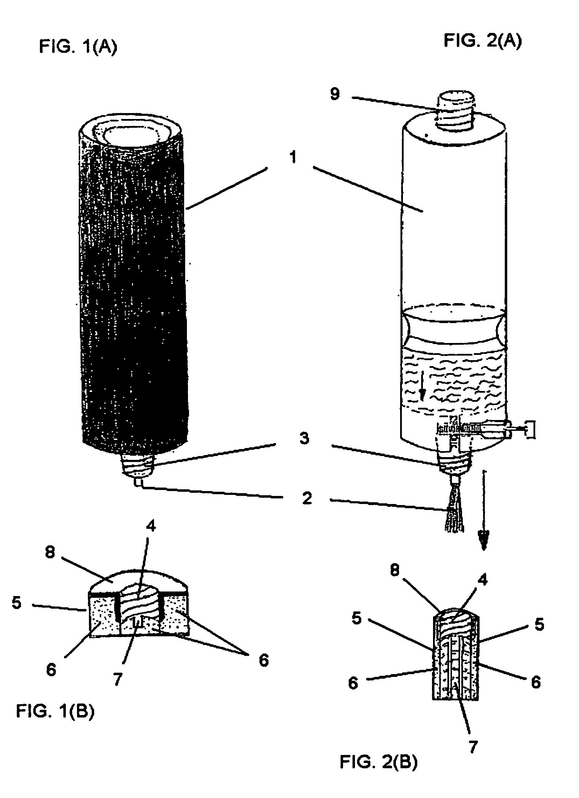

Figur 1(A) zeigt schematisch eine Abgabeeinrichtung (auch Auftragsgerät genannt) zum Abgeben und Auftragen eines in einem Behälter 1 der Abgabeeinrichtung enthaltenen Fluids durch ein Auftragsorgan 2, das in diesem Fall durch eine Düse gebildet ist. Die Düse befindet sich mittig in einem ein Verbindungsteil 3 bildenden zylindrischen Ansatz, der mit einem Außengewinde versehen ist.

Figur 1(B) zeigt ein dafür vorgesehenes Behältnis. Das Behältnis weist ein Gehäuse 5 auf, in dem sich vorzugsweise ein poröses oder saugfähiges Material 6 (zum Beispiel in Form eines Schwamms) befindet, das mit einem Verdünnungs- oder Lösungsmittel (siehe unten) getränkt ist. Das Material 6 dient auf diese Weise zum Halten des Verdünnungs- oder Lösungsmittels und in geringerem Umfang auch dazu, dass sich dieses weitgehend gleichmäßig auf dem Auftragsorgan niederschlägt.

Gegebenenfalls könnte auf das Material 6 auch verzichtet und das Verdünnungs- oder Lösungsmittel in Form einer freien Flüssigkeit direkt in das Behältnis eingebracht werden. Dies gilt insbesondere dann, wenn das Behältnis in seinem Inneren so ausgeformt ist, dass die Gefahr eines unbeabsichtigten Auslaufens des Verdünnungs- oder Lösungsmittel relativ gering ist.

Das Behältnis ist mit einem Verbindungsteil 4 in Form einer Gewindeverbindung, vorzugsweise in Form eines Sacklochs 4 mit einem Innengewinde versehen, das so bemessen ist, dass damit das Behältnis auf das Verbindungsteil 3, d.h. den mit einem korrespondierenden Außengewinde versehenen zylindrischen Ansatz 3 der Abgabeeinrichtung aufgeschraubt werden kann.

Das Sackloch 4 ist entweder direkt in das poröse oder saugfähige Material 6 eingebracht, oder es ist durch eine Gewindehülse gebildet, die an einer Stirnfläche 8 des Gehäuses 5 befestigt ist.

Am Grund des Sacklochs 4 befindet sich ein freier Raum oder eine Ausnehmung 7, der/die kein Material 6 enthält und so bemessen ist, dass dieser ausreichend Platz für die Düse 2 der Abgabeeinrichtung bietet, vorzugsweise ohne das Material 6 zu berühren, wenn das Behältnis auf den zylindrischen Ansatz 3 aufgedreht ist.

Dies hat den Vorteil, dass das Verdünnungs- oder Lösungsmittel ausschließlich durch Verdampfung aus dem Material 6 und anschließenden Niederschlag auf die Düse 2 gelangt. Wenn die Düse 2 hingegen das Material 6 berührt, so besteht die Gefahr, das das in der Düse 2 vorhandene Fluid durch den Kapillareffekt aus der Düse 2 abgesaugt wird, so dass, insbesondere dann, wenn dabei weiteres Fluid aus der Abgabeeinrichtung nachfließt, das Material 6 relativ schnell mit Fluid verschmutzt wird. Dies gilt natürlich auch für Auftragsorgane anderer Art.

Der freie Raum 7 wird entweder in Form einer Ausnehmung direkt in das poröse oder saugfähige Material 6 eingebracht, oder dadurch geschaffen, dass das Material 6 nur in die Seitenbereiche des Gehäuses 5 eingelegt wird.

Das Verbindungsteil 4, das gemäß Figur 1(B) in Form einer Schraubverbindung (d.h. Sackloch 4 mit Innengewinde) ausgebildet ist, kann zur Befestigung des Behältnisses an der Abgabeeinrichtung alternativ auch in Form einer Bajonett- oder Steckverbindung o.ä. vorgesehen sein. Generell sollte das Verbindungsteil 4 einen so weit wie möglich luftdichten Abschluss zwischen Auftragsorgan 2 und dem Innenraum bzw. der Ausnehmung 7 des Gehäuses 5 ermöglichen.

Vorzugsweise ist die bereits erwähnte Stirnfläche 8 des Gehäuses 5, in der sich die Öffnung des Sacklochs 4 befindet, hinsichtlich ihres Verlaufes, ihrer Form und ihrer Abmessungen zumindest im Wesentlichen an den Verlauf, die Form und die Abmessungen der Fläche der Abgabeeinrichtung angepasst, auf die die Stirnfläche 8 beim Aufschrauben des Behältnisses auf den zylindrischen Ansatz 3 der Abgabeeinrichtung zu liegen kommt, um eine abdichtende Wirkung zwischen beiden zu erzielen. Vorzugsweise ist die Stirnfläche 8 zu diesem Zweck zum Beispiel mit einer Gummiauflage versehen.

Ferner kann die Stirnfläche 8 zur Verminderung der Verdunstung von in dem Behältnis vorhandenen Verdünnungs- oder Lösungsmittel bei Nichtgebrauch des Behältnisses auch möglichst plan ausgebildet und/oder mit einer Gummiauflage versehen sein, so dass das Behältnis mit seiner Öffnung auf eine Unterlage gelegt werden kann, um dadurch die Öffnung des Verbindungsteils 4 in dem Gehäuse 5 zu verschließen.

Alternativ dazu kann zu diesem Zweck auch ein Deckel (nicht dargestellt) vorgesehen sein, der anstelle des zylindrischen Ansatzes 3 auf das Sackloch 4 aufgeschraubt oder aufgesetzt wird, um das Behältnis zumindest weitgehend luftdicht zu verschließen und eine Verdunstung des Verdünnungs- oder Lösungsmittels zu vermeiden, wenn das Behältnis nicht benötigt wird (zum Beispiel während der Verwendung der Abgabeeinrichtung).

Wie oben bereits erwähnt wurde, ist das poröse oder saugfähige Material 6 mit einem Verdünnungs- oder Lösungsmittel getränkt. Dieses wird in Abhängigkeit von dem in dem Behälter 1 der betreffenden Abgabeeinrichtung vorhandenen, aufzutragenden Fluid so gewählt, dass es dieses Fluid flüssig halten kann und damit ein Eintrocknen des Fluids verhindert, wenn es mit dem Fluid und insbesondere mit den an dem Auftragsorgan 2 vorhandenen Fluidresten in Berührung kommt, d.h. sich auf dem Auftragsorgan 2 durch Verdampfung aus dem Material 6 niederschlägt.

Besonders bevorzugt werden bei allen Ausführungsformen als Verdünnungs- oder Lösungsmittel stets diejenigen Mittel verwendet, die auch Bestandteil des aus der betreffenden Abgabeeinrichtung aufzutragenden Fluids sind, und mit denen dieses flüssig gehalten wird.

Nachdem also die Abgabeeinrichtung benutzt worden ist, wird das Behältnis auf den zylindrischen Ansatz 3 aufgeschraubt, so dass die Düse 2 in der beschriebenen Weise in den freien Raum 7 eintritt oder von diesem zumindest weitgehend umgeben ist. Das in dem Material 6 vorhandene Verdünnungs- oder Lösungsmittel lagert sich insbesondere durch Verdampfen aus dem Material 6 und anschließenden Niederschlag an der Öffnung der Düse 2 ab und verhindert damit ein Eintrocknen des dort vorhandenen Fluids. Die Abgabeeinrichtung bleibt damit über sehr lange Zeit einsatzbereit, auch nachdem sie das erste Mal benutzt bzw. angebrochen worden ist.

Figur 2(A) zeigt schematisch eine weitere Abgabeeinrichtung (auch Auftragsgerät genannt) zum Abgeben und Auftragen eines in einem Behälter 1 der Abgabeeinrichtung enthaltenen Fluids durch ein Auftragsorgan, das in diesem Fall durch einen Pinsel 2 gebildet ist. Der Pinsel 2 befindet sich mittig in einem zylindrischen Ansatz 3, der mit einem Außengewinde versehen ist und wiederum ein Verbindungsteil an der Abgabeeinrichtung bildet.

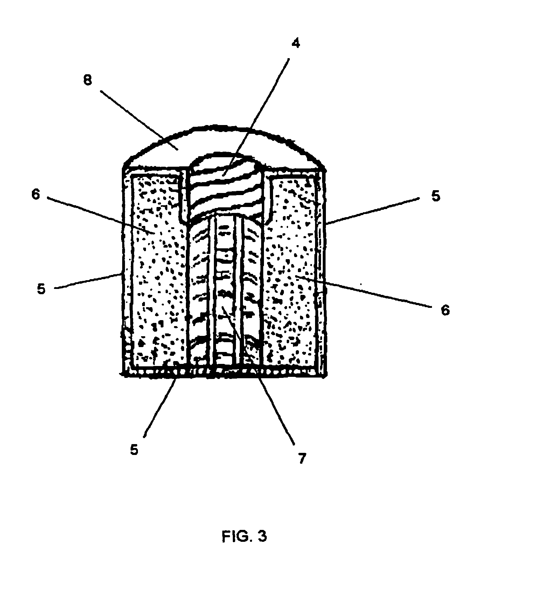

Figur 2(B) zeigt ein dafür vorgesehenes Behältnis, das in Figur 3 in vergrößerter Darstellung wiedergegeben ist.

Das Behältnis weist wiederum ein Gehäuse 5 auf, in dem sich ein poröses oder saugfähiges Material 6 befindet. Das Behältnis ist wiederum mit einem Verbindungsteil in Form eines Sacklochs 4 mit einem Innengewinde (Gewindeverbindung) versehen, das so bemessen ist, dass damit das Behältnis auf den zylindrischen Ansatz 3 der Abgabeeinrichtung aufgedreht werden kann.

Zur Aufbewahrung bei der Anwendung der Abgabeeinrichtung kann das Behältnis vorzugsweise auf einen Gewindeansatz 9 an der Abgabeeinrichtung (siehe Figur 2(A)) luftdicht aufgeschraubt werden. In diesem Fall wird der oben erwähnte Deckel für das Behältnis nicht benötigt.

Das Sackloch 4 in dem Behältnis setzt sich wiederum in Form eines freien Raums 7 fort, der kein Material 6 enthält und so bemessen ist, dass er den Pinsel 2 an der Abgabeeinrichtung aufnehmen kann, und zwar aus den oben genannten Gründen vorzugsweise ohne das Material 6 zu berühren, wenn das Behältnis auf den zylindrischen Ansatz 3 aufgedreht wird.

Das Sackloch 4 ist entweder direkt in das poröse oder saugfähige Material 6 eingebracht, oder es ist durch eine Gewindehülse gebildet, die von einer Stirnfläche 8 des Gehäuses 5 ausgeht. Der freie Raum 7 ist wiederum direkt in das poröse oder saugfähige Material 6 eingebracht oder von diesem Material 6 freigehalten, so dass der Pinsel 2 ohne Berührung mit dem Material 6 in diesen Raum 7 zu liegen kommen kann.

Das Verbindungsteil 4 zur Befestigung des Behältnisses an der Abgabeeinrichtung kann auch bei dieser Ausführungsform alternativ als Bajonett- oder Steckverbindung o.ä. vorgesehen sein.

Die Stirnfläche 8 des Gehäuses 5, in der sich die Öffnung des Sacklochs 4 befindet, ist wiederum vorzugsweise hinsichtlich ihres Verlaufes, ihrer Form und ihrer Abmessungen zumindest im Wesentlichen an den Verlauf, die Form und die Abmessungen der Fläche der Abgabeeinrichtung angepasst, auf die die Stirnfläche 8 beim Aufschrauben des Behältnisses auf den zylindrischen Ansatz 3 der Abgabeeinrichtung zu liegen kommt, um eine abdichtende Wirkung zwischen beiden zu erzielen. Zu diesem Zweck kann die Stirnfläche 8 wiederum mit einer Gummiauflage versehen sein.

Alternativ dazu kann die Stirnfläche auch, wie oben beschrieben wurde, plan und/oder mit einer Gummiauflage versehen sein, um eine abdichtende Auflage des Behältnisses auf einer Unterlage zu ermöglichen und somit ein Verdunsten des in dem Behältnis vorhandenen Verdünnungs- oder Lösungsmittel aus dem Behältnis zu vermeiden, wenn es nicht benötigt wird.

Hinsichtlich der Funktion und Wirkung des porösen oder saugfähigen Materials 6 und der Art des Verdünnungs- oder Lösungsmittels gilt das gleiche, was oben in Bezug auf die erste Ausführungsform beschrieben wurde.

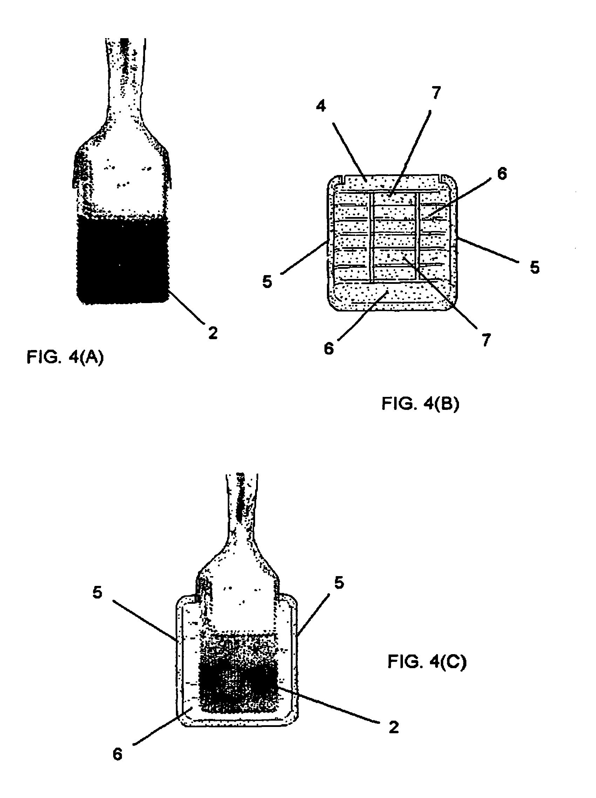

Figur 4(A) zeigt eine Abgabeeinrichtung in Form eines (Hand-) Pinsels, dessen Auftragsorgan durch die Pinsel-Borsten 2 gebildet ist. Das in Figur 4(B) gezeigte Behältnis ist hinsichtlich seiner Form und Abmessungen an das Borstenteil 2 des Pinsels angepasst, so dass dieses gemäß Figur 4(C) in das Behältnis eingesetzt werden kann.

Auch diese dritte Ausführungsform eines erfindungsgemäßen Behältnisses weist ein Gehäuse 5 auf, in dem sich ein poröses oder saugfähiges Material 6 befindet, das mit einem wie oben erläutert gewählten Verdünnungs- oder Lösungsmittel für das mit dem Pinsel aufzutragende Fluid getränkt ist.

Das Gehäuse 5 weist ein Verbindungsteil in Form einer Öffnung 4 auf, durch die das Borstenteil 2 des Pinsels in eine entsprechend der Form und Abmessungen des Borstenteils 2 bemessene Ausnehmung 7 in dem Material 6 eingesetzt werden kann, vorzugsweise ohne das Material 6 zu berühren, und wobei die Öffnung 4 so ausgebildet ist, dass sie den an das Borstenteil 2 angrenzenden Abschnitt des Pinsels vorzugsweise durch Klemmung oder Arretierung hält. Gegebenenfalls kann der Pinsel mit korrespondierenden Arretierungselementen versehen sein.

Somit ist das Borstenteil 2 des Pinsels gemäß Figur 4(C) von dem Material 6 umgeben, so dass sich das in diesem vorhandene Verdünnungs- oder Lösungsmittel nach Verdampfen aus dem Material 6 an dem Borstenteil 2 des Pinsels niederschlägt und dadurch ein Eintrocknen des an dem Borstenteil 2 vorhandenen Fluids verhindert.

Das erfindungsgemäße Behältnis kann bei allen Ausführungsformen in einfacher Weise durch das eine Öffnung in dem Gehäuse 5 bildende Verbindungsteil 4 mit Verdünnungs- oder Lösungsmittel nachgefüllt werden.

## Patentansprüche

1. Behältnis für ein Auftragsorgan einer Abgabeeinrichtung oder eines Auftragsgerätes für Fluide, zur Aufnahme des Auftragsorgans (2) bei Nichtgebrauch , wobei das Behältnis ein Gehäuse (5) aufweist, das
- ein Verbindungsteil (4) zur zumindest im Wesentlichen luftdichten Verbindung des Behältnisses mit einem korrespondierenden Verbindungsteil (3) an der Abgabeeinrichtung oder dem Auftragsgerät,
- ein poröses oder saugfähiges Material (6), das mit einem Verdünnungs- oder Lösungsmittel für das Fluid getränkt ist, und
- einen freien Raum oder eine Ausnehmung (7) in dem porösen oder saugfähigen Material (6) zur zumindest im Wesentlichen berührungsfreien Aufnahme des Auftragsorgans (2) aufweist, wobei
- das Verdünnungs- oder Lösungsmittel so gewählt ist, dass es sich nach Verdampfung durch Niederschlag auf dem Auftragsorgan (2) ablagert.

2. Behältnis nach Anspruch 1,
bei dem das Verbindungsteil (4) des Gehäuses (5) durch ein mit einem Innengewinde versehenes Sackloch gebildet ist, das zur schraubbaren Befestigung des Behältnisses an einem in Form eines mit einem Außengewinde versehenen zylindrischen Ansatzes ausgebildeten, korrespondierenden Verbindungsteil (3) an einer Abgabeeinrichtung oder einem Auftragsgerät ausgebildet ist.

3. Behältnis nach Anspruch 1,
bei dem das Verbindungsteil (4) des Gehäuses (5) zur steckbaren Verbindung oder Arretierung des Behältnisses an einem korrespondierenden Verbindungsteil (3) einer Abgabeeinrichtung oder eines Auftragsgerätes ausgebildet ist.

4. Behältnis nach Anspruch 1,
bei dem der das Verbindungsteil (4) umgebende Bereich des Gehäuses (5) zur abdichtenden Anlage an einem ein Auftragsorgan (2) umgebenden Bereich einer Abgabeeinrichtung ausgebildet ist.

5. Behältnis nach Anspruch 1,
bei dem der das Verbindungsteil (4) umgebende Bereich des Gehäuses (5) zur abdichtenden Auflage des Behältnisses auf einer Unterlage bei dessen Nichtgebrauch ausgebildet ist.

6. Behältnis nach Anspruch 1,
mit einem Deckel, der zum zumindest im Wesentlichen luftdichten Verschließen des Behältnisses mit dem Verbindungsteil (4) verbunden werden kann.

7. Behältnis nach Anspruch 1,
für eine Abgabeeinrichtung in Form eines Pinsels, wobei das Auftragsorgan (2) durch ein Borstenteil des Pinsels gebildet ist, wobei das Gehäuse (5) ein Verbindungsteil (4) in Form einer Öffnung aufweist, durch die das Borstenteil des Pinsels in die Ausnehmung (7) einsetzbar ist, und wobei die Öffnung so ausgebildet ist, dass sie einen an das Borstenteil angrenzenden Abschnitt des Pinsels durch Klemmung oder Arretierung hält.

8. Abgabeeinrichtung oder Auftragsgerät für Fluide, mit einem Auftragsorgan (2) zur Abgabe und zum Auftragen des in einem Behälter (1) enthaltenen Fluids, mit einem Verbindungsteil (3) zur Befestigung eines Behältnisses nach einem der vorhergehenden Ansprüche für das Auftragsorgan (2) mit einem korrespondierenden Verbindungsteil (4).

## Claims

1. Receptacle for an application means of a dispense device or of an applicator for fluids, for receiving the application means (2) when not in use, wherein the receptacle comprises a casing (5) which comprises:
- a connecting component (4) for at least substantially air-tight connecting the receptacle with a corresponding connecting component (3) at the dispense device or the applicator,
- a porous or absorbent material (6) which is soaked with a diluting agent or a solvent for the fluid, and
- a free space or a recess (7) in the porous or absorbent material (6) for the at least substantially contactless reception ofthe application means (2), wherein
- the diluting agent or solvent is selected such that after vaporization it deposits by precipitation on the application means (2).

2. Receptacle according to claim 1,
wherein the connecting component (4) of the casing (5) is provided by a blind hole with an internal screw thread, which is provided for attaching by screwing the receptacle at a corresponding connecting component (3) of a dispense device or an applicator, wherein the corresponding connecting component (3) is provided in form of a cylindrical projection with an external screw thread.

3. Receptacle according to claim 1,
wherein the connecting component (4) of the casing (5) is provided for connecting by plugging or locking the receptacle at a corresponding connecting component (3) of a dispense device or an applicator.

4. Receptacle according to claim 1,
wherein the region of the casing (5) surrounding the connecting component (4) is provided for sealingly abutting on a region surrounding an application means (2) of a dispense device.

5. Receptacle according to claim 1,
wherein the region of the casing (5) surrounding the connecting component (4) is provided for sealingly resting the receptacle on a support when not in use.

6. Receptacle according to claim 1,
comprising a cover which can be connected with the connecting component (4) for at least substantially air-tight closure ofthe receptacle.

7. Receptacle according to claim 1,
for a dispense device in the form of a brush, wherein the application means (2) is provided by brush-bristles of the brush, wherein the casing (5) comprises a connecting component (4) in form of an opening, through which the brush-bristles can be inserted into the recess (7), and wherein the opening is formed such that it holds a part ofthe brush adjacent to the brush-bristles by clamping or locking.

8. Dispense device or applicator for fluids, with an application means (2) for dispensing and applying fluid contained in a vessel (1), comprising a connecting component (3) for attaching a receptacle according to one of the preceding claims for the application means (2), wherein the receptacle comprises a corresponding connecting component (4).

## Revendications

1. Réceptacle pour des moyens d'application d'un dispositif de distribution ou d'un applicateur de fluides, destiné à recevoir les moyens d'application (2) lorsqu'il n'est pas en utilisation, le réceptacle comprenant un carter (5) qui comprend:
- un composant de raccordement (4) pour raccorder le réceptacle de manière au moins sensiblement étanche à l'air à un composant de raccordement correspondant (3) au niveau du dispositif de distribution ou de l'applicateur,
- un matériau poreux ou absorbant (6) qui est plongé dans un agent de dilution ou un solvant du fluide, et
- un espace libre ou un évidement (7) dans le matériau poreux ou absorbant (6) pour la réception de manière au moins sensiblement sans contact des moyens d'application (2), dans lequel
- l'agent de dilution ou le solvant est sélectionné de telle sorte qu'après vaporisation il se dépose par précipitation sur les moyens d'application (2).

2. Réceptacle selon la revendication 1,
dans lequel le composant de raccordement (4) du carter (5) est fourni par un trou borgne ayant un filetage interne, qui est fourni pour fixation par vissage du réceptacle au niveau d'un composant de raccordement correspondant (3) d'un dispositif de distribution ou d'un applicateur, dans lequel le composant de raccordement correspondant (3) est fourni sous la forme d'une saillie cylindrique ayant un filetage externe.

3. Réceptacle selon la revendication 1,
dans lequel le composant de raccordement (4) du carter (5) est fourni pour raccordement par bouchage ou blocage du réceptacle au niveau d'un composant de raccordement correspondant (3) d'un dispositif de distribution ou d'un applicateur.

4. Réceptacle selon la revendication 1,
dans lequel la zone du carter (5) entourant le composant de raccordement (4) est fournie pour venir en butée de manière étanche sur une zone entourant des moyens d'application (2) d'un dispositif de distribution.

5. Réceptacle selon la revendication 1,
dans lequel la zone du carter (5) entourant le composant de raccordement (4) est fournie pour appuyer de manière étanche le réceptacle sur un support lorsqu'il n'est pas en utilisation.

6. Réceptacle selon la revendication 1,
comprenant un couvercle qui peut être raccordé au composant de raccordement (4) pour fermer le réceptacle de manière sensiblement étanche à l'air.

7. Réceptacle selon la revendication 1,
destiné à un dispositif de distribution ayant la forme d'une brosse, dans lequel les moyens d'application (2) sont fournis par des poils de brosse de la brosse, dans lequel le carter (5) comprend un composant de raccordement (4) sous la forme d'une ouverture, à travers laquelle les poils de brosse peuvent être insérés dans l'évidement (7), et dans lequel l'ouverture est formée de telle sorte qu'elle maintient une partie de la brosse adjacente aux poils de brosse par serrage ou blocage.

8. Dispositif de distribution ou applicateur de fluides, ayant des moyens d'application (2) pour distribuer et appliquer du fluide contenu dans un récipient (1), comprenant un composant de raccordement (3) pour fixation d'un réceptacle selon l'une des revendications précédentes destiné aux moyens d'application (2), dans lequel le réceptacle comprend un composant de raccordement correspondant (4).
